# EUROPEAN PATENT APPLICATION

(11) **EP 0 972 778 A1**
(43) Date of publication of application: **19.01.2000**
(21) Application number: 99302111.2
(22) Date of filing: 18.03.1999
(51) Int. Cl.: C07H 17/08

(54) **Erythromycin derivatives and methods for the preparation thereof**

(30) Priority: 15.07.1998 IL 0125372
(71) Applicant: Chemagis Ltd., Bnei Brak (IL)
(72) Inventor: Nadaka, Vladimir, Lod 71338 (IL); Kaspi, Joseph, Givataim 53201 (IL)
(74) Representative: Ablewhite, Alan James

(57) **Abstract**

The invention provides the compound, 6-O-methylerythromycin A 9-hydrazone, of formula I

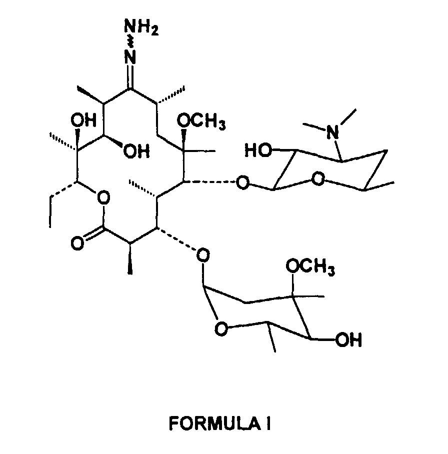

## Description

### Field of The Invention

The present invention relates to 6-O-methylerythromycin 9-hydrazone, a novel 6-O-methylerythromycin A derivative, and a method for its preparation, said derivative being useflul as an intermediate for the preparation of the antibiotic drug 6-O-methylerythromycin A (clarithromycin).

### Description of Prior Art

Erythromycin A is regarded as one of the safest antibiotics. However, the compound loses its antibacterial activity rapidly under acidic conditions due to intermolecular cyclization. One approach to improve the stability is preventing this cyclization by methylation of the hydroxy group at the 6-position. 6-O-methylerythromycin A shows an excellent efficacy for treatment of bacterial infections by oral administration. There are several known methods for the preparation of 6-O-methylerythromycin A, for example, the methods for the selective methylation of the hydroxy group at 6-position of erythromycin A derivatives which comprise converting erythromycin A derivatives having the protected hydroxy group at the 2'-position and the protected dimethylamino group at the 3'-position into the various kinds of the substituted oxime derivatives, followed by carrying out methylation of the hydroxy group at 6-position, removal of the protecting groups, deoximation at the 9-position and regeneration of the dimethylamino group at the 3'-position to give 6-O-methylerythromycin A, which methods are described in European Patent 195,960 and European Patent 158,467. Similarly, U.S. Patent 4,990,602 discloses an improved method for the selective methylation of the hydroxy group at the 6-position of erythromycin A 9-oxime derivatives and is based on the protection of the hydroxy groups at 2' - and 4'' - positions by the substituted silyl groups and the hydroxy group in 9-oxime moiety by an alkoxyalkyl group which are easily removed after the methylation of the hydroxy group at the 6-position.

Although it is possible to methylate the 6-hydroxy group selectively by these methods, the methods described in said European patents require complicated procedures such as catalytic reduction for removal of the protecting groups after methylation, and the method of said US patent requires purification by column chromatography.

### SUMMARY OF THE INVENTION

With this state of the art in mind, there is now provided according to the present invention 6-O-methylerythromycin A 9-hydrazone which is useful as an intermediate for preparing 6-O-methylerythromycin A and methods for the preparation and use thereof.

More specifically, the present invention provides novel 6-O-methylerythromycin A 9-hydrazone (hereinafter referred to as compound I) represented by the formula I.

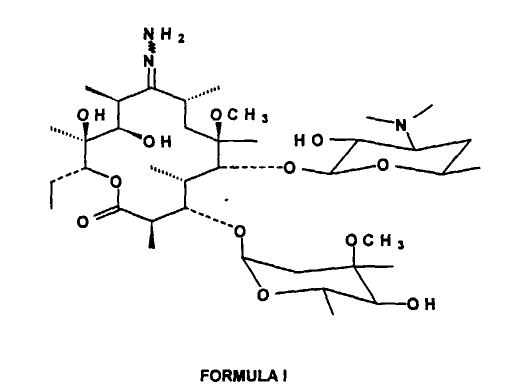

Another object of the present invention is to provide a method for preparing 6-O-methylerythromycin A 9-hydrazone from 6-O-methylerythromycin A 9-azine derivatives comprising, reacting compounds of formula II : 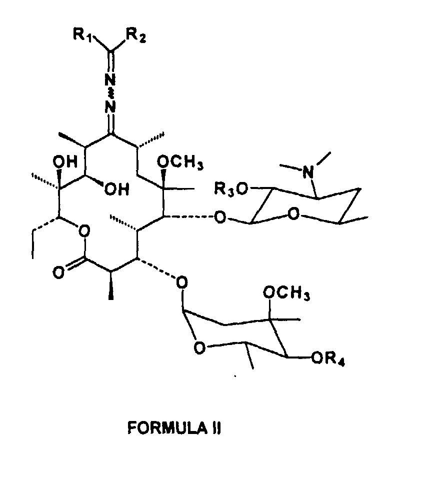 wherein R₁ is a hydrogen atom or an alkyl group having 1 to 6 carbon atoms, R₂ is an alkyl group having 1 to 6 carbon atoms or an unsubstituted or substituted aryl group, or R₁ and R₂ together form a linear or branched chain alkylene ring having 3 to 10 carbon atoms, an arylenedialkylene group in which the alkylene moiety has 2 to 3 carbon atoms or an arylendiakylene group in which the alkylene moieties have 1 to 2 carbon atoms, R₃ and R₄ are independently hydrogens or substituted silyl groups, with hydrazine in the presence of an acid in a solvent and optionally removing the silyl protecting groups.

In a preferred embodiment of the present invention said hydrazine is added in the form of an aqueous solution selected from the group consisting of hydrazine hydrate and hydrazine hydrochloride.

In a further preferred embodiment more than 1.5 equivalents of said hydrazine are used per equivalent of compound of formula II.

In an even more preferred embodiment said acid is selected from the group consisting of formic acid, acetic acid, propionic acid and hydrochloric, wherein said acid is present in an amount providing a pH of 5.5 to 8.0, wherein the solvent is an aqueous alcohol, wherein said alcohol is selected from the group consisting of methanol, ethanol and 2-propanol and wherein the amount of the solvent is from 0.9 to 10 ml per 1g of compound of formula II.

In a another aspect of the invention there is provided a method for preparing 6-O-methylerythromycin A 9-oxime from 6-O-methylerythromycin A 9-hydrazine which comprises reacting 6-O-methylerythromycin A 9-hydrazine with hydroxylamine in the presence of an acid in a solvent.

In a preferred embodiment said hydroxylamine is added in the form of an aqueous solution or hydroxylamine hydrochloride, wherein at least 1.5 equivalents of said hydroxylamine is used per equivalent of 6-O-methylerythromycin A 9-hydrazine, wherein said acid is selected from the group consisting of formic acid, acetic acid, propionic acid and hydrochloride acid, wherein said acid is present in an amount providing a pH of 5.5 to 8.0, wherein the solvent is an alcohol, wherein said alcohol is selected from the group consisting of methanol, ethanol and 2-propanol, and wherein the amount of the solvent is from 0.9 to 10 ml per 1g of 6-O-methylerythromycin A 9-hydrazine.

In a further embodiment, the above method includes an additional step of a deoximation of 6-O-methylerythromycin A 9-oxime to give 6-O-methylerythromycin A.

The compound having formula II can be converted into compound I, for example, by the following method. The protecting groups of the hydroxy groups at the 2'-and 4'' positions (R₃ and R₄) of the compounds of formula II can be easily removed in an aqueous alcohol medium in the presence of an acid at room temperature to give 9-azine 6-O-methylerythromycin A derivatives represented by the general formula III : 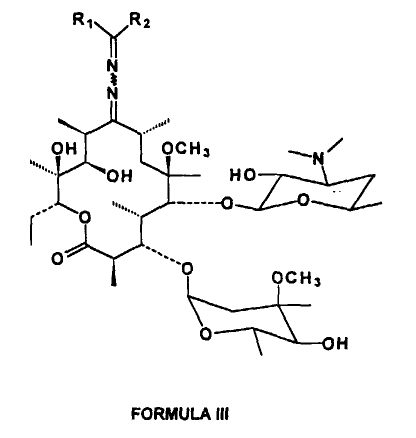 wherein R₁ and R₂ are as defined in formula II. Methods for preparing the compounds of formula II and formula III have been described by the present inventors in IL Specification No. 124084.

Compounds of formula III thus obtained can be easily converted into compound I by reaction with hydrazine in the presence of an acid in an alcohol. For example, formic acid and hydrazine are added to a suspension of the compound of formula III in methanol, and the mixture is stirred at 55°-62°C to give compound I. Formic acid is added in amount to adjust the pH of the reaction solution from 5.5 to 8.0, and preferably from 7.0 to 8.0. The amount of hydrazine is more than 1.5 equivalents relative to the compound of formula III. The hydrazine can be added in the form of an aqueous solution, hydrazine hydrate or hydrazine hydrochloride or sulfate. Since the progress of the reaction can be monitored by using thin layer chromatography or high prformance liquid chromotography, the reaction may be stopped after the disappearance of the starting material. Alternatively, since the production of hydrazone from azine is carried out under acidic conditions, this process can be done conveniently at the same time as the above removal of the protecting groups. For example, an acid (e.g., formic acid, acetic acid, and the like) is added to a solution of compound of formula II in an aqueous alcohol (e.g., methanol, ethanol, and the like) and the mixture is stirred at room temperature to remove the silyl groups at the 2'-and 4'' positions of the compound. Then hydrazine (in any of the forms mentioned above) is added to the mixture, and the reaction mixture is heated at 55°-62°C to give compound I. The acid is added in an amount to adjust the pH of the reaction solution from 5.5 to 8.0, and preferably from 7.0 to 8.0. The amount of hydrazine is more than 1.5 equivalents and preferably from 6 to 10 molar equivalents relative to the compound of formula II. The amount of reaction solvent is preferably from 0.9 to 10 ml and more preferably from 1 to 3 ml relative to 1g of the compound of formula II. The reaction temperature may be chosen from room temperature to the reflux temperature of the solvent, and preferably from 55° to 62°C. Although compound I of the present invention may exist as two isomers (syn- and anti-forms for a hydrazone group at the 9-position), for the purpose of the present invention, this compound may exist as either of the isomers or as a mixture thereof.

Therefore, the present invention can provide 6-O-methylerythromycin A in high yield. Namely, compound I can be led to 6-O-methylerythromycin A, for example, by the following method:

The compound I is reacted with hydroxylamine in an alcohol (e.g., methanol, ethanol and the like) in the presence of an acid at heating to give 6-O-methylerythromycin A 9- oxime. In order to obtain 6-O-methylerythromycin A 9-oxime in high yield, the amount of hydroxylamine must be more than 1.5 equivalents and preferably from 6 to 10 molar equivalents relative to the compound I. The acid is added in an amount to adjust the pH of the reaction solution from 5.5 to 8.0, and preferably from 6.0 to 7.0. The amount of the reaction solvent is preferably from 0.9 to 10 ml, and more preferably from 1 to 5 ml relative to 1g of the compound I. The reaction temperature may be chosen from room temperature to the reflux temperature of the solvent, and preferably from 55° to 62°C. In the reaction the hydroxylamine can be added in the form of an aqueous solution or hydroxylamine hydrochloride. The conclusion of the reaction can be recognized by determining the disappearance of the compound I using thin layer chromatography or high performance liquid chromatography. 6-O-methlerythromycin A 9-oxime thus obtained can be easily converted into 6-O-methylerythromycin A by deoximation. Deoximation is carried out in accordance with standard procedures well known in the art (see e.g., patent US 4,990,602). Briefly, the 6-O-methylerythromycin A 9-oxime is reacted with sodium hydrogen sulfite (or sodium metabisulfite) in the presence of formic acid in aqueous alcohol (e.g., ethanol) at reflux. The solution is cooled, alkalinized and precipitated with aqueous sodium hydroxide solution. The precipitate formed in the above reaction is collected by filtration, washed and recrystallized from alcohol.

The present invention will be illustrated by Examples which show the method for preparing compound I and Example B which shows the method for preparing 6-O-methylerythromycin A.

While the invention will now be described in connection with certain preferred embodiments in the following examples so that aspects thereof may be more fully understood and appreciated, it is not intended to limit the invention to these particular embodiments. On the contrary, it is intended to cover all alternatives, modifications and equivalents as may be included within the scope of the invention as defined by the appended claims. Thus, the following examples which include preferred embodiments will serve to illustrate the practice of this invention, it being understood that the particulars shown are by way of example and for purposes of illustrative discussion of preferred embodiments of the present invention only and are presented in the cause of providing what is believed to be the most useful and readily understood description of formulation procedures as well as of the principles and conceptual aspects of the invention.

### Example A

### Preparation of 6-O-methylerythromycin A 9-cyclohexanoneazine from 2', 4''-O-bis(tri-methylsilyl)-6-O-methylerythromycin A 9-cyclohexanoneazine

To a solution of 20 g of 2', 4''-O-bis(trimethylsilyl)-6-O-methylerythromycin A 9-cyclohexanoneazine in 50 ml of ethanol/water (10/1) was added 2.8 ml of 99% formic acid, and the mixture was stirred at ambient temperature for 2 hours. Then the reaction mixture was diluted with 60 ml of water, made basic (pH 11) with 45% aqueous sodium hydroxide solution at 15-20°C and extracted with ethyl acetate. The organic layer was washed with water and a saturated aqueous sodium chloride solution and dried over anhydrous magnesium sulfate. The solvent was evaporated reduced pressure to give 16.2g of 6-O-methylerythromycin A 9-cyclohexanoneazine as a foam. m.p. 176 - 179°C (recrystallized from ethanol) [α]_{D}²⁵ - 144.2°(c=1.0, CHCl₃).

### Example 1

### Preparation of 6-O-methylerythromycin A 9-hydrazone from 6-O-methylerythromycin A 9-cyclohexanoneazine

To a suspension of 10 g of 6-O-methylerythromycin A 9-cyclohexanoneazine (prepared according to Example A), in 45 ml of methanol, there was added 0.9 ml of 99% formic acid and 5 ml of hydrazine monohydrate, and the reaction mixture was stirred at 60-62°C for 3 hours. Then, the rection solution was diluted with 65 ml of water, made basic (pH 11) with 45% aqueous sodium hydroxide solution with ice-cooling, stirred at 5°C for two hours and kept at this temperature overnight. The precipitate which formed was collected by filtration and recrystallized from ethanol to give 7.33g of 6-O-methylerythromycin A 9-hydrazone.
m.p. 186-189°C (dec) [α]_{D}²⁷ - 53.5° (c=1.0, CHCl₃).
¹H-NMR (CDCl₃) : δ(ppm) = 0.83[3H, t, CH₃ (C-15)], 2.29[6H, s, 3'-N(CH₃)₂], 3.20(3H, s, 6-OCH₃), 3.33 (3H, s, 3''-OCH₃).
¹³C-NMR(CDCl₃) : δ(ppm) = 9.45(C-17), 10.61(C-15), 14.55(C-20), 40.29[3'-N(CH₃)₂], 49.45(3''-OCH₃), 51.75(6-OCH₃), 96.35(C-1''), 102.33(C-1'), 167.67(C-9), 175.05(C-1).
MS(FAB) : m/z 763(MH⁺).

### Example 2

### Preparation of 6-O-methylerythromycin A 9-hydrazone from 2', 4''-O-bis(trimethylsilyl)-6-O-methylerythromycin A 9-cyclohexanoneazine

To a mixture of 20 g of 2'4''-O-bis(trimethylsilyl)-6-O-methylerythromycin A 9-cyclohexanoneazine, 50 ml of ethanol and 3 ml of water was added 2.8 ml of 99% formic acid, and the mixture was stirred at ambient temperature for 3 hours. Then, 11.2 ml of hydrazine monohydrate were added to the solution, and the mixture (pH 8) was stirred at 60-62°C for 3 hours. The reaction mixture was diluted with 50 ml of water, made basic (pH 11) with 45% aqueous sodium hydroxide solution with ice-cooling, stirred at 5°C for two hours and kept at this temperature overnight. A colorless precipitate was collected by filtration, washed with water and dried at 50°C to give 9.7 g of 6-O-methylerythromycin A 9-hydrazone, which was identical to the compound obtained in Example 1.

### Example 3

### Preparation of 6-O-methylerythromycin A 9-hydrazone from 2', 4''-O-bis (trimethylsilyl)-6-O-methylerythromycin A 9-acetoneazine

To a mixture of 1.25 g of 2', 4''-O-bis(trimethylsilyl)-6-O-methylerythromycin A 9-acetoneazine, 10 ml of ethanol and 0.5 ml of water was added 0.3 ml of 99% formic acid, and the mixture was stirred at ambient temperature for 3 hours. Then, 0.7 ml of hydrazine monohydrate was added to the solution and the mixture (pH 8) was stirred at 60-62°C for one hour. The solution was cooled, diluted with 20 ml of water, and 45% aqueous sodium hydroxide solution was added drop wise to obtain pH 11. The mixture was stirred at 5°C for two hours and kept at this temperature overnight. A colorless precipitate was collected by filtration, washed with water and dried at 50°C to give 0.83 g or 6-O-methylerythromycin A 9-hydrazone, which was identical to the compound obtained in Example 1.

### Example 4

### Preparation of 6-O-methylerythromycin A 9-oxime from 6-O-methylerythromycin A 9-hydrazone

To a suspension of 5 g of 6-O-methylerythromycin A 9-hydrazone, obtained in Example 2, in 25 ml of methanol were added at 60°C 2.5 ml of 50% aqueous hydroxylamine solution and a solution of 3.0 g of hydroxylamine hydrochloride in 3 ml of water. The reaction mixture (pH 6) was stirred at 60°C for 25.5 hours. Then, the mixture was cooled to ambient temperature, diluted with 25 ml of water, made basic (pH 11) with 5% aqueous sodium hydroxide solution and extracted with ethyl acetate. The organic layer was washed with water and a saturated aqueous sodium chloride solution and dried over anhydrous magnesium sulfate. The solvent was removed to dryness under reduced pressure to give 4.7g of 6-O-methylerythromycin A 9-oxime.
m.p. 248° - 251°C. (melted at 169° - 185°C, and melted again at 248° - 251°C)(recrystallized from ethanol - petroleum ether).
[α]_{D}²² - 91.3° (c=1.0, CHCl₃).
¹H - NMR(CDCl₃) : δ(ppm) = 0.83[3H, t, CH₃(C -15)], 2.36[6H, s, 3' - N(CH₃)₂], 3.10(3H, s, 6 - OCH₃), 3.33(3H, s, 3'' - OCH₃).
¹³C-NMR(CDCl₃) : δ(ppm) = 9.21(C - 17), 10.61 (C - 15), 14.97(C - 20), 40.40 [3' - N(CH₃)₂], 49.48(3'' - OCH₃), 51.19(6-OCH₃), 96.03(C-1''), 102.70(C-1'), 170.36(C-9), 175.67(C-1).
MS(FAB) : m/z 764(MH⁺).

### Example B

### Preparation of 6-O-methylerythromycin A from 6-O-methylerythromycin A 9 - oxime.

2 g of 6-O-methylerythromycin A 9-oxime, obtained in Example 4, and 1.1 g of sodium metabisulfite in 20ml of ethanol/water (1/1) were added with 0.25 ml of 99% formic acid, and the mixture was refluxed for 100 minutes. The reaction mixture was diluted with 30 ml of water, made basic (pH 11) with 5% aqueous sodium hydroxide solution and stirred with ice - cooling for two hours. The precipitate which formed was collected by filtration and recrystallized from ethanol to give 1.45 g of 6-O-methylerythromycin A.
m.p. 222° - 225°C.
[α]_{D}²⁰ - 93.3°(c 1.0, CHCl₃).

It will be evident to those skilled in the art that the invention is not limited to the details of the foregoing illustrative examples and that the present invention may be embodied in other specific forms without departing from the essential attributes thereof, and it is therefore desired that the present embodiments and examples be considered in all respects as illustrative and not restrictive, reference being made to the appended claims, rather than to the foregoing description, and all changes which come within the meaning and range of equivalency of the claims are therefore intended to be embraced therein.

## Claims

1. The compound, 6-O-methylerythromycin A 9-hydrazone, of formula I

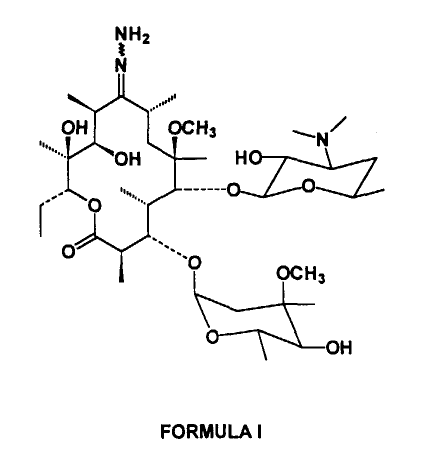

2. A method for preparing 6-O-methylerythromycin A 9-hydrazone from 6-O-methylerythromycin A 9-azine derivatives comprising, reacting compounds of formula II : 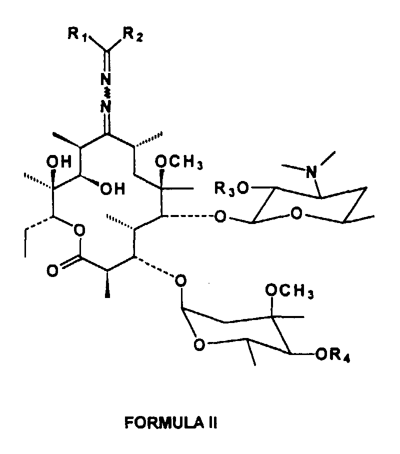 wherein R₁ is a hydrogen atom or an alkyl group having 1 to 6 carbon atoms, R₂ is an alkyl group having 1 to 6 carbon atoms or an unsubstituted or substituted aryl group, or R₁ and R₂ together form a linear or branched chain alkylene ring having 3 to 10 carbon atoms, an arylenedialkylene group in which the alkylene moiety has 2 to 3 carbon atoms or an arylendiakylene group in which the alkylene moities have 1 to 2 carbon atoms, R₃ and R₄ are independently hydrogens or substituted silyl groups, with hydrazine in the presence of an acid in a solvent and optionally removing the silyl protecting groups.

3. A method of claim 2 wherein said hydrazine is added in the form of an aqueous solution selected from the group consisting of hydrazine hydrate and hydrazine hydrochloride.

4. A method of claim 2 wherein more than 1.5 equivalents of said hydrazine are used per equivalent of compound of formula II.

5. A method according to claim 2, wherein said acid is selected from the group consisting of formic acid, acetic acid, propionic acid and hydrochloric acid.

6. A method according to claim 2, wherein said acid is present in an amount providing a pH of 5.5 to 8.0.

7. A method according to claim 2, wherein the solvent is an aqueous alcohol.

8. A method according to claim 7, wherein said alcohol is selected from the group consisting of methanol, ethanol and 2-propanol.

9. A method according to claim 2, wherein the amount of the solvent is from 0.9 to 10 ml per 1g of compound of formula II.

10. A method for preparing 6-O-methylerythromycin A 9-oxime from 6-O-methylerythromycin A 9-hydrazone which comprises reacting 6-O-methylerythromycin A 9-hydrazone with hydroxylamine in the presence of an acid in a solvent.

11. A method of claim 10 wherein said hydroxylamine is added in the form of an aqueous solution or hydroxylamine hydrochloride.

12. A method of claim 10 wherein at least 1.5 equivalents of said hydroxylamine is used per equivalent of 6-O-methylerythromycin A 9-hydrazone.

13. A method according to claim 10, wherein said acid is selected from the group consisting of formic acid, acetic acid, propionic acid and hydrochloride acid.

14. A method according to claim 10, wherein said acid is present in an amount providing a pH of 5.5 to 8.0.

15. A method according to claim 10, wherein the solvent is an alcohol.

16. A method according to claim 15, wherein said alcohol is is selected from the group consisting of methanol, ethanol and 2-propanol.

17. A method according to claim 10, wherein the amount of the solvent is from 0.9 to 10 ml per 1g of 6-O-methylerythromycin A 9-hydrazone.

18. A method according to claim 10, which includes an additional step of a deoximation of 6-O-methylerythromycin A 9-oxime to give 6-O-methylerythromycin A.
